# EUROPEAN PATENT APPLICATION

(11) **EP 4 667 452 A1**
(43) Date of publication of application: **24.12.2025**
(21) Application number: 25153577.9
(22) Date of filing: 23.01.2025
(51) Int. Cl.: C07C 69/618, C07C 229/44, C07C 237/20, C07C 317/48, C07D 295/155

(54) **BLUE LIGHT BLOCKING COMPOUND, BLUE LIGHT BLOCKING COMPOSITION, USE OF BLUE LIGHT BLOCKING COMPOUND, USE OF BLUE LIGHT BLOCKING COMPOSITION AND METHOD FOR PREPARING BLUE LIGHT BLOCKING COMPOUND**

(30) Priority: 18.06.2024 US 202463661088 P; 05.12.2024 TW 113147204
(71) Applicant: Visco Vision Inc., Taoyuan City 33341 (TW)
(72) Inventor: CHANG, Chih-Yi, 33341 Taoyuan City (TW); CHIANG, Ching-Hsueh, 33341 Taoyuan City (TW); CHANG, Hsiao-Mei, 33341 Taoyuan City (TW)
(74) Representative: dompatent

(57) **Abstract**

A blue light blocking compound and a use thereof, a blue light blocking composition and a use thereof, and a method for preparing a blue light blocking compound are provided. The blue light blocking compound is represented by the following formula 1. In the formula 1, R₁ and R₈ are each independently H, a substituted or unsubstituted alkyl group, a substituted or unsubstituted alkenyl group, halogen, N(R^{1a})₂, OR^{1b}, SR^{1c}, SO₂R^{1d}, COR^{1e}, NO₂, CN, SOR^{1h}, a five-membered heterocycle, a six-membered heterocycle, or a fused ring structure formed by connecting R₁ and R₈.

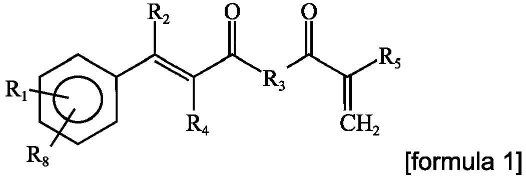

## Description

### BACKGROUND

### Technical Field

The present disclosure relates to a blue light blocking compound, a blue light blocking composition, a use of a blue light blocking compound, a use of a blue light blocking composition, and a method for preparing a blue light blocking compound.

### Description of the Related Art

Visible light is electromagnetic radiation with a wavelength between 380 nm and 780 nm, and electromagnetic radiation with a wavelength between 380 nm and 500 nm can be called blue light in a broad sense. Too much exposure to blue light may cause harmful effects on health, such as damage to cells in the retina, macular degeneration, eye fatigue, glare and accelerated age-related eye problems. Therefore, it is very important to prevent too much blue light from entering the eyes.

Using an ophthalmic device which can block blue light (also known as blue light blocking ophthalmic device) is one of the methods to effectively avoid harmful effects on health caused by blue light. When the blue light-blocking ophthalmic device is placed on the surface of the user's eye, it can block (e.g., absorb or reflect) at least part of the blue light, thereby reducing the amount of blue light entering the eye. The blue light blocking ophthalmic device is manufactured by incorporating a blue light blocking compound into the material of the ophthalmic device. However, the reactivity and miscibility of existing blue light blocking compound with the material of the ophthalmic device are poor, resulting an insufficient blue light blocking effect.

### SUMMARY

The present disclosure provides a blue light blocking compound, a blue light blocking composition including the blue light blocking compound, a use of a blue light blocking compound in manufacturing an ophthalmic device, an optical film, a screen protector, a display, etc., a use of a blue light blocking composition in manufacturing an ophthalmic device, an optical film, a screen protector, a display, etc., and a method for preparing a blue light blocking compound. The blue light blocking compound shows good reactivity and miscibility, and thus products such as ophthalmic devices, optical films, screen protectors or displays formed by the blue light blocking compound of the present disclosure can exhibit good blue light blocking effect.

According to an embodiment, a blue light blocking compound is provided. The blue light blocking compound is represented by the following formula 1. In the formula 1, R₁ and R₈ are each independently H, a substituted or unsubstituted alkyl group, a substituted or unsubstituted alkenyl group, an alkenyl group substituted with halogen, halogen, N(R^{1a})₂, OR^{1b}, SR^{1c}, SO₂R^{1d}, COR^{1e}, NO₂, CN, SOR^{1h}, a five-membered heterocycle, a six-membered heterocycle, or a fused ring structure formed by connecting R₁ and R₈, wherein R^{1a} and R^{1b} are each independently H, a substituted or unsubstituted alkyl group, a substituted or unsubstituted alkenyl group, or a substituted or unsubstituted phenyl group; R^{1d} is a substituted or unsubstituted alkyl group, a substituted or unsubstituted alkenyl group or OH; R^{1e} is H, OH, a substituted or unsubstituted alkyl group, a substituted or unsubstituted alkenyl group or -O-R^{1f}; R^{1c}, R^{1f} and R^{1h} are each independently a substituted or unsubstituted alkyl group, or a substituted or unsubstituted alkenyl group; R₂ is H or a substituted or unsubstituted C₁~C₄ alkyl group; R3 is X-(CₙH₂ₙOₘ)ₚ, wherein X is O, NH, S or SO₂, m is 1 to 4, n is 1 to 4, p is 1 to 4; R₄ is CN or C(O)R₈, wherein R₆ is H, a substituted or unsubstituted alkyl group, a substituted or unsubstituted alkenyl group, R₃-C(O)-CCH₂R₅ or O-R^{1g,} R^{1g} is a substituted or unsubstituted C₁~C₄ alkyl group, or a substituted or unsubstituted C₁~C₄ alkenyl group; R₅ is H or a substituted or unsubstituted C₁~C₄ alkyl group.

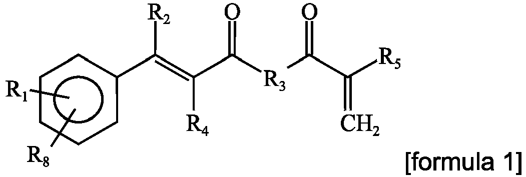

According to another embodiment, a blue light blocking composition is provided. The blue light blocking composition includes a blue light blocking compound represented by the above formula 1, and a polymerizable monomer. In the formula 1, R₁ and R₈ are each independently H, a substituted or unsubstituted alkyl group, a substituted or unsubstituted alkenyl group, halogen, N(R^{1a})₂, OR^{1b}, SR^{1c}, SO₂R^{1d}, COR^{1e}, NO₂, CN, SOR^{1h}, a five-membered heterocycle, a six-membered heterocycle, or a fused ring structure formed by connecting R₁ and R₈, wherein R^{1a} and R^{1b} are each independently H, a substituted or unsubstituted alkyl group, a substituted or unsubstituted alkenyl group, or a substituted or unsubstituted phenyl group; R^{1d} is a substituted or unsubstituted alkyl group, a substituted or unsubstituted alkenyl group or OH; R^{1e} is H, OH, a substituted or unsubstituted alkyl group, a substituted or unsubstituted alkenyl group or -O-R^{1f}; R^{1c}, R^{1f} and R^{1h} are each independently a substituted or unsubstituted alkyl group, or a substituted or unsubstituted alkenyl group; R₂ is H or a substituted or unsubstituted C₁~C₄ alkyl group; R₃ is X-(CₙH₂ₙOₘ)ₚ, wherein X is O, NH, S or SO2, m is 1 to 4, n is 1 to 4, p is 1 to 4; R₄ is CN or C(O)R₆, wherein R₆ is H, a substituted or unsubstituted alkyl group, a substituted or unsubstituted alkenyl group, R₃-C(O)-CCH₂R₅ or O-R^{1g}, R^{1g} is a substituted or unsubstituted C₁~C₄ alkyl group, or a substituted or unsubstituted C₁~C₄ alkenyl group; R₅ is H or a substituted or unsubstituted C₁~C₄ alkyl group.

According to yet another embodiment, a use of a blue light blocking compound is provided. The blue light blocking compound is used in manufacturing an ophthalmic device.

According to yet another embodiment, a use of a blue light blocking composition is provided. The blue light blocking composition is used in manufacturing an optical film, a screen protector, a display or an ophthalmic device. The blue light blocking composition includes a blue light blocking compound represented by the above formula 1, and a polymerizable monomer. In the formula 1, R₁ and R₈ are each independently H, a substituted or unsubstituted alkyl group, a substituted or unsubstituted alkenyl group, halogen, N(R^{1a})₂, OR^{1b}, SR^{1c}, SO₂R^{1d}, COR^{1e}, NO₂, CN, SOR^{1h}, a five-membered heterocycle, a six-membered heterocycle, or a fused ring structure formed by connecting R₁ and R₈, wherein R^{1a} and R^{1b} are each independently H, a substituted or unsubstituted alkyl group, a substituted or unsubstituted alkenyl group, or a substituted or unsubstituted phenyl group; R^{1d} is a substituted or unsubstituted alkyl group, a substituted or unsubstituted alkenyl group or OH; R^{1e} is H, OH, a substituted or unsubstituted alkyl group, a substituted or unsubstituted alkenyl group or -O-R^{1f}; R^{1c}, R^{1f} and R^{1h} are each independently a substituted or unsubstituted alkyl group, or a substituted or unsubstituted alkenyl group; R₂ is H or a substituted or unsubstituted C₁~C₄ alkyl group; R₃ is X-(CₙH₂ₙOₘ)ₚ, wherein X is O, NH, S or SO2, m is 1 to 4, n is 1 to 4, p is 1 to 4; R₄ is CN or C(O)R₆, wherein R₆ is H, a substituted or unsubstituted alkyl group, a substituted or unsubstituted alkenyl group, R₃-C(O)-CCH₂R₅ or O-R^{1g}, R^{1g} is a substituted or unsubstituted C₁~C₄ alkyl group, or a substituted or unsubstituted C₁~C₄ alkenyl group; R₅ is H or a substituted or unsubstituted C₁~C₄ alkyl group.

According to yet another embodiment, a method for preparing a blue light blocking compound is provided. The method includes: providing a compound represented by the following formula 2; hydrolyzing the compound to obtain an intermediate product; esterifying the intermediate product to obtain the blue light blocking compound. In the formula 2, R₁ and R₈ are each independently H, a substituted or unsubstituted alkyl group, a substituted or unsubstituted alkenyl group, halogen, N(R^{1a})₂, OR^{1b}, SR^{1c}, SO₂R^{1d}, COR^{1e}, NO₂, CN, SOR^{1h}, a five-membered heterocycle, a six-membered heterocycle, or a fused ring structure formed by connecting R₁ and R₈; R^{1a} and R^{1b} are each independently H, a substituted or unsubstituted alkyl group, a substituted or unsubstituted alkenyl group, or a substituted or unsubstituted phenyl group; R^{1d} is a substituted or unsubstituted alkyl group, a substituted or unsubstituted alkenyl group or OH; R^{1e} is H, OH, a substituted or unsubstituted alkyl group, a substituted or unsubstituted alkenyl group or -O-R^{1f}; R^{1c}, R^{1f} and R^{1h} are each independently a substituted or unsubstituted alkyl group, or a substituted or unsubstituted alkenyl group; R₂ is H or a substituted or unsubstituted C₁~C₄ alkyl group; R₄' is CN or C(O)R₆', wherein R₆' is H, a substituted or unsubstituted alkyl group, a substituted or unsubstituted alkenyl group, or O-R^{1j}, R^{1j} is a substituted or unsubstituted C₁~C₄ alkyl group, or a substituted or unsubstituted C₁~C₄ alkenyl group.

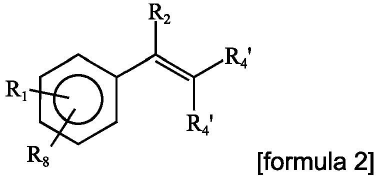

The above and other embodiments of the disclosure will become better understood with regard to the following detailed description of the non-limiting embodiment(s).

### DETAILED DESCRIPTION

As used in the specification, the term "ophthalmic device" refers to a device placed on the surface of the eye, which may or may not directly contact the surface of the eye. The Ophthalmic device can have functions such as vision correction, disease treatment, drug delivery, and appearance change. Ophthalmic devices include, but are not limited to, soft contact lenses and rigid contact lenses. Soft contact lenses include, but are not limited to, hydrogel contact lenses and silicone hydrogel contact lenses. As used in the specification, the term "hydrogel contact lens" refers to a contact lens whose structure does not contain silicone groups. As used in the specification, the term "silicone hydrogel contact lens" refers to a contact lens whose structure contains silicone groups.

As used in the specification, the term "use in manufacturing an ophthalmic device" may mean that the blue light blocking compound or the blue light blocking composition according to the present disclosure is added to the material of the ophthalmic device, or may mean that the blue light blocking compound or the blue light blocking composition according to the present disclosure is attached to the surface of the ophthalmic device. The present disclosure is not limited thereto.

In the specification, if it is not specified whether a group is substituted, the group may refer to a substituted or unsubstituted group. For example, "alkyl group" may refer to a substituted or unsubstituted alkyl group, "alkenyl group" may refer to a substituted or unsubstituted alkenyl group, and "phenyl group" may refer to a substituted or unsubstituted phenyl group. In addition, when "Cₓ" is used to describe a group, it means that the main chain of the group has x carbon atoms.

In the specification, the structure of compound may be represented by a skeleton formula. This representation can omit carbon atoms, hydrogen atoms, and carbon-hydrogen bonds. Of course, if the functional group is clearly shown in the structure, the structure will be as drawn. In the specification, if the benzene ring includes a line extending from the inside to the outside of the benzene ring and one end of this line is connected to a group, it means that the substitution site of this group at the benzene ring is uncertain and structures formed by substituting atoms at different sites on the benzene ring with this group are within the scope of the present disclosure.

The present disclosure provides a blue light blocking compound represented by the following formula 1.

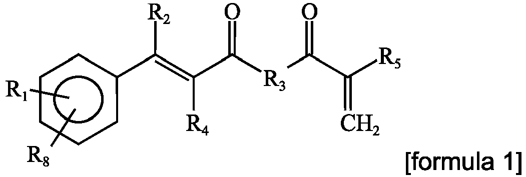

In the formula 1, R₁ and R₈ are each independently H, a substituted or unsubstituted alkyl group, a substituted or unsubstituted alkenyl group, halogen, N(R^{1a})₂, OR^{1b}, SR^{1c}, SO₂R^{1d}, COR^{1e}, NO₂, CN, SOR^{1h}, a five-membered heterocycle, a six-membered heterocycle, or a fused ring structure formed by connecting R₁ and R₈, R^{1a} and R^{1b} are each independently H, a substituted or unsubstituted alkyl group, a substituted or unsubstituted alkenyl group, or a substituted or unsubstituted phenyl group, R^{1d} is a substituted or unsubstituted alkyl group, a substituted or unsubstituted alkenyl group or OH, R^{1e} is H, OH, a substituted or unsubstituted alkyl group, a substituted or unsubstituted alkenyl group or -O-R^{1f}; R^{1c}, R^{1f} and R^{1h} are each independently a substituted or unsubstituted alkyl group, or a substituted or unsubstituted alkenyl group; R₂ is H or a substituted or unsubstituted C₁~C₄ alkyl group; R₃ is X-(CₙH₂ₙOₘ)ₚ, X is O, NH, S or SO₂, m is 1 to 4, n is 1 to 4, p is 1 to 4; R₄ is CN or C(O)R₆, R₆ is H, a substituted or unsubstituted alkyl group, a substituted or unsubstituted alkenyl group, R₃-C(O)-CCH₂R₅ or O-R^{1g}; R₅ is H or a substituted or unsubstituted C₁~C₄ alkyl group; R^{1g} is a substituted or unsubstituted C₁~C₄ alkyl group, or a substituted or unsubstituted C₁~C₄ alkenyl group.

In an embodiment, in the formula 1, R₁ and R₈ are each independently H, an unsubstituted alkyl group, an alkyl group substituted with halogen, halogen, N(R^{1a})₂, OR^{1b}, SR^{1c}, SO₂R^{1d}, COR^{1e}, NO₂, CN, SOR^{1h}, a five-membered heterocycle, a six-membered heterocycle, or a fused ring structure formed by connecting R₁ and R₈, wherein R^{1a} and R^{1b} are each independently H, a substituted or unsubstituted alkyl group, a substituted or unsubstituted alkenyl group, or a substituted or unsubstituted phenyl group, R^{1d} is a substituted or unsubstituted alkyl group, a substituted or unsubstituted alkenyl group or OH, R^{1e} is H, OH, a substituted or unsubstituted alkyl group, a substituted or unsubstituted alkenyl group or -O-R^{1f}; R^{1c}, R^{1f} and R^{1h} are each independently a substituted or unsubstituted alkyl group, or a substituted or unsubstituted alkenyl group; R₂ is H or a substituted or unsubstituted C₁~C₄ alkyl group; R₃ is X-(CₙH₂ₙOₘ)ₚ, X is O, NH, S or SO₂, m is 1 to 4, n is 1 to 4, p is 1 to 4; R₄ is CN or C(O)R₆, R₆ is H, a substituted or unsubstituted alkyl group, a substituted or unsubstituted alkenyl group, R₃-C(O)-CCH₂R₅ or O-R^{1g}; R₅ is H or a substituted or unsubstituted C₁~C₄ alkyl group; R^{1g} is a substituted or unsubstituted C₁~C₄ alkyl group, or a substituted or unsubstituted C₁~C₄ alkenyl group.

In an embodiment, in the formula 1, R₁ and R₈ are each independently H, an unsubstituted C₁~C₄ alkyl group, a C₁~C₄ alkyl group substituted with halogen, an unsubstituted C₁~C₄ alkenyl group, a C₁~C₄ alkenyl group substituted with halogen, halogen, N(R^{1a})₂, OR^{1b}, SR^{1c}, SO₂R^{1d}, COR^{1e}, NO₂, CN, SOR^{1h}, a five-membered heterocycle, a six-membered heterocycle, or a fused ring structure formed by connecting R₁ and R₈.

In an embodiment, in the formula 1, R₁ and R₈ are each independently H, an unsubstituted C₁~C₄ alkyl group, a C₁~C₄ alkyl group substituted with halogen, an unsubstituted C₁~C₄ alkenyl group, a C₁~C₄ alkenyl group substituted with halogen, halogen, N(R^{1a})₂, OR^{1b}, SR^{1c}, SO₂R^{1d}, COR^{1e}, NO₂, CN, SOR^{1h}, a heterocycle represented by the following formula 3, a heterocycle represented by the following formula 4 or a heterocycle represented by the following formula 5. The a heterocycle represented by the following formula 3, the heterocycle represented by the following formula 4 and the heterocycle represented by the following formula 5 are connected to the benzene ring in formula 1 through their N atoms. In an embodiment, R₁ can be connected to R₈ to form a fused ring structure represented by the following formula 6 or formula 7 (the fused ring structure represented by the formula 6 or formula 7 contains the benzene ring shown in formula 1).

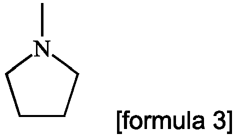

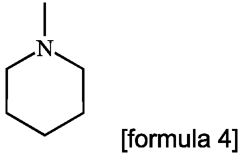

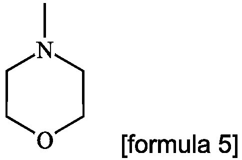

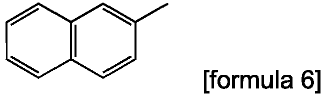

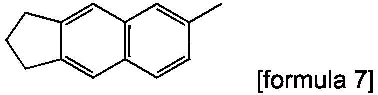

In an embodiment, in the formula 1, R₁ and R₈ are each independently H, an unsubstituted alkyl group, an alkyl group substituted with halogen, an unsubstituted alkenyl group, an alkenyl group substituted with halogen, halogen, N(R^{1a})₂, OR^{1b}, SOR^{1h}, a five-membered heterocycle, a six-membered heterocycle, or a fused ring structure formed by connecting R₁ and R₈, wherein R^{1a} and R^{1b} are each independently H, a substituted or unsubstituted alkyl group, a substituted or unsubstituted alkenyl group, or a substituted or unsubstituted phenyl group, R^{1h} is a substituted or unsubstituted alkyl group.

In an embodiment, in the formula 1, R₁ and R₈ are each independently H, an unsubstituted C₁~C₄ alkyl group, a C₁~C₄ alkyl group substituted with halogen, an unsubstituted C₁~C₄ alkenyl group, a C₁~C₄ alkenyl group substituted with halogen, halogen, N(R^{1a})₂, OR^{1b}, SOR^{1h}, a five-membered heterocycle, a six-membered heterocycle, or a fused ring structure formed by connecting R₁ and R₈, wherein R^{1a} and R^{1b} are each independently H, a substituted or unsubstituted alkyl group, a substituted or unsubstituted alkenyl group, or a substituted or unsubstituted phenyl group, R^{1h} is a substituted or unsubstituted alkyl group.

In an embodiment, in the formula 1, R₁ and R₈ are each independently H, a C₁~C₄ alkyl group substituted with F, a C₁~C₄ alkenyl group substituted with F, halogen, N(R^{1a})₂, OR^{1b}, SOR^{1h}, a five-membered heterocycle, a six-membered heterocycle, or a fused ring structure formed by connecting R₁ and R₈, wherein R^{1a} and R^{1b} are each independently H, a substituted or unsubstituted alkyl group, a substituted or unsubstituted alkenyl group, or a substituted or unsubstituted phenyl group, R^{1h} is a substituted or unsubstituted alkyl group.

In an embodiment, in the formula 1, R₃ is X-(CₙH₂ₙOₘ)ₚ, wherein X is O or NH; m is 1 to 3; n is 1 to 3; p is 1 to 3.

In an embodiment, in the formula 1, R₃ is X-(CₙH₂ₙOₘ)ₚ, wherein X is O or NH; m is 1 to 2; n is 1 to 2; p is 1 to 2.

In an embodiment, in the formula 1, R₄ is C(O)R₆, wherein R₆ is H, a substituted or unsubstituted alkyl group, a substituted or unsubstituted alkenyl group, R₃-C(O)-CCH₂R₅ or O-R^{1g}; R₅ and R^{1g} are defined as above.

In an embodiment, in the formula 1, R₄ is C(O)R₆, wherein R₆ is an alkyl group, an alkenyl group or R₃-C(O)-CCH₂R₅ or O-R^{1g}; R₅ and R^{1g} are defined as above.

In an embodiment, the "a substituted or unsubstituted alkyl group" in the above formula 1 can refer to a substituted or unsubstituted C₁~C₄ alkyl group. In an embodiment, the "a substituted or unsubstituted alkenyl group" in the above formula 1 can refer to a substituted or unsubstituted C₁~C₄ alkenyl group.

In an embodiment, the blue light blocking compound can ne represented by any one of the following formulas 1-1 to 1-9.

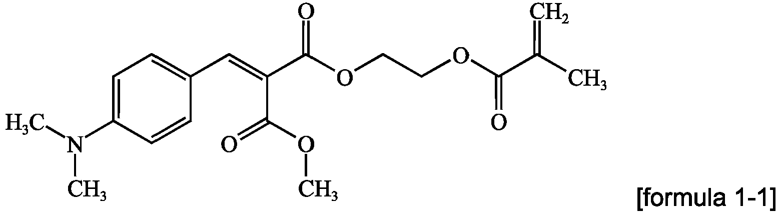

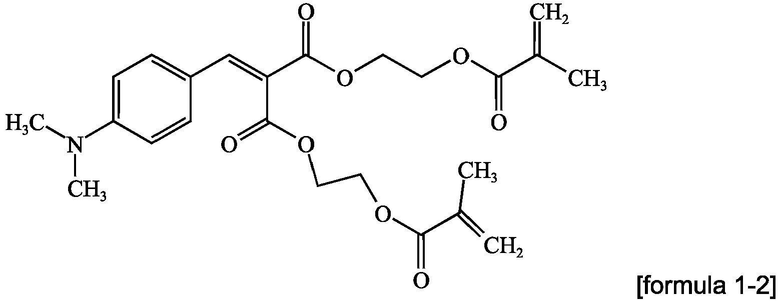

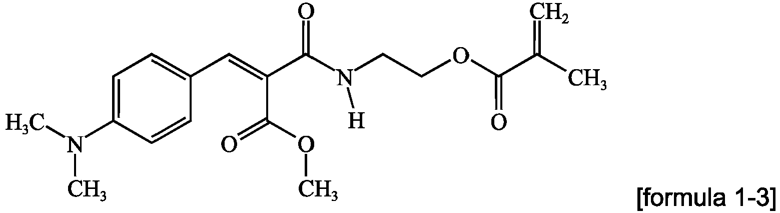

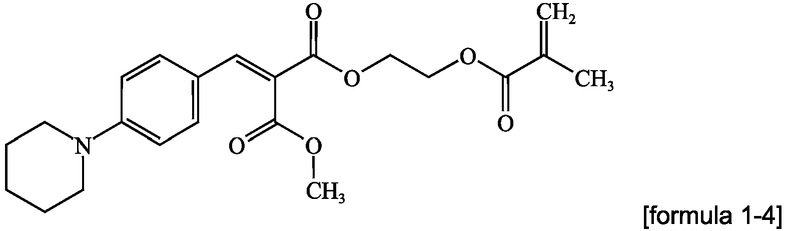

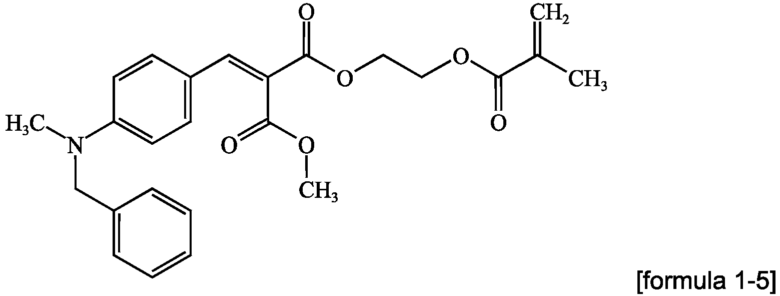

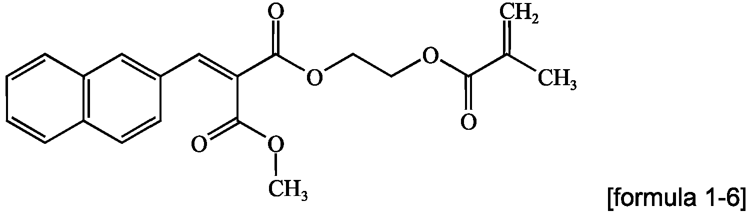

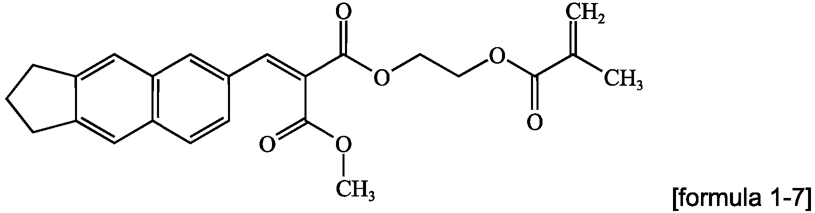

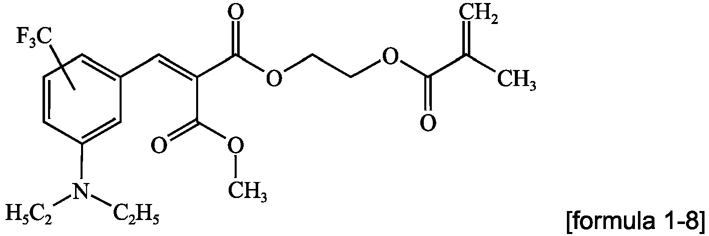

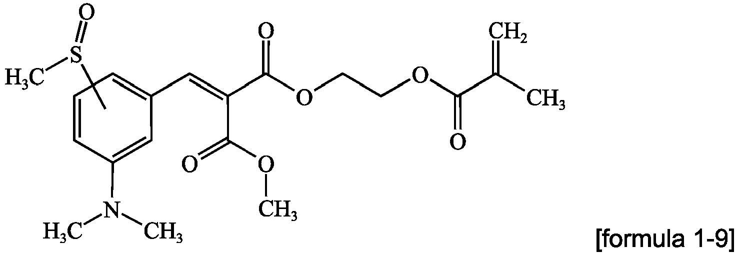

A method for manufacturing the blue light blocking compound according to the present disclosure can include the following steps.

A compound represented by the following formula 2 is provided. In the formula 2, R₁, R₈ and R₂ are defined as above. In the formula 2, R₄' is CN or C(O)R₆', wherein R₆' is H, a substituted or unsubstituted alkyl group, a substituted or unsubstituted alkenyl group, or O-R^{1j}, R^{1j} is a substituted or unsubstituted C₁~C₄ alkyl group, or a substituted or unsubstituted C₁~C₄ alkenyl group. The compound is hydrolyzed to obtain an intermediate product. In an embodiment, the compound, an alkaline reagent, a solvent and water can be mixed to undergo a hydrolysis reaction, and then be extracted with ethyl acetate to obtain the intermediate product. The alkaline reagent can be at least one selected from a group consisting of sodium carbonate, potassium carbonate, potassium hydroxide and sodium hydroxide. The solvent can be at least one selected from a group consisting of acetone, tetrahydrofuran, methanol, ethanol, n-propanol, isopropanol, methylene chloride and ethyl acetate.

For example, the water used in the aforementioned step can be pure water, filtered water, distilled water, electrolyzed water, etc.

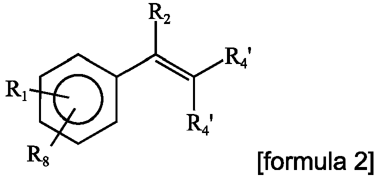

In an embodiment, the compound represented by the following formula 2 can be the compound represented by the following formula 2-1.

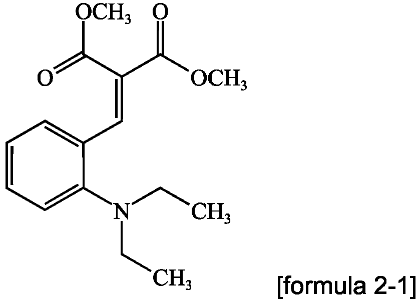

In an embodiment, the compound represented by the following formula 2 can be the compound represented by the following formula 2-2.

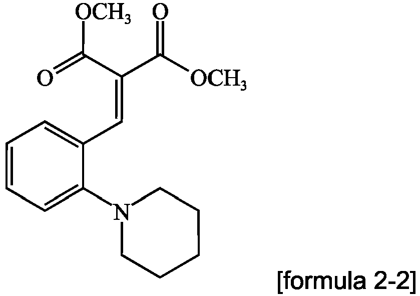

In an embodiment, the compound represented by the following formula 2 can be the compound represented by the following formula 2-3.

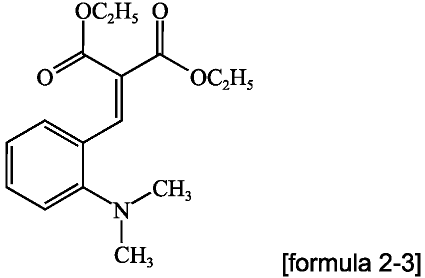

Then, the intermediate product is esterified to obtain the blue light blocking compound represented by the formula 1. In an embodiment, the intermediate product, a hydrophilic compound and a solvent can be mixed to undergo an esterification reaction to obtain the blue light blocking compound represented by the formula 1. The hydrophilic compound is an acrylic ester compound containing a hydroxyl group. The hydrophilic compound can be at least one selected from a group consisting of 2-hydroxyethyl methacrylate (HEMA), 4-hydroxybutyl acrylate (HBA), glycidyl methacrylate (GMA), hydroxypropyl methacrylate (HPMA), 2-hydroxyisopropyl methacrylate (HIPMA), and hydroxybutyl methacrylate (HBMA). The solvent can be at least one selected from a group consisting of acetone, tetrahydrofuran, methanol, ethanol, n-propanol, isopropanol, methylene chloride and ethyl acetate.

The blue light blocking compound of the present disclosure can be used to manufacture an ophthalmic device, an optical film, a screen protector or a display. The blue light blocking compound of the present disclosure can be used to prepare a blue light blocking composition, and the blue light blocking composition can be used to manufacture an ophthalmic device, an optical film, a screen protector or a display. The blue light blocking composition can include a blue light blocking compound and a polymerizable monomer. In an embodiment, the polymerizable monomer can include a monomer containing an alkenyl group or a hydrophilic monomer containing an alkenyl group and hydrophilic.

In an embodiment, the blue light blocking compound of the present disclosure can be used to prepare a blue light blocking composition, and the blue light blocking composition can be used to manufacture an ophthalmic device. The blue light blocking composition for manufacturing an ophthalmic device includes the blue light blocking compound and the polymerizable monomer. The polymerizable monomer can include at least one of a hydrophilic monomer, a first siloxane monomer and a second siloxane monomer. The polymerizable monomer can be at least one selected from a group consisting of a hydrophilic monomer, a first siloxane monomer and a second siloxane monomer. The first siloxane monomer is different from the second siloxane monomer. In an embodiment, the polymerizable monomer includes the hydrophilic monomer. In an embodiment, the polymerizable monomer consists of the hydrophilic monomer. In an embodiment, the polymerizable monomer includes the hydrophilic monomer and the first siloxane monomer. In an embodiment, the polymerizable monomer consists of the hydrophilic monomer and the first siloxane monomer. In an embodiment, the polymerizable monomer includes the hydrophilic monomer and the second siloxane monomer. In an embodiment, the polymerizable monomer consists of the hydrophilic monomer and the second siloxane monomer. In an embodiment, the polymerizable monomer includes the hydrophilic monomer, the first siloxane monomer and the second siloxane monomer. In an embodiment, the polymerizable monomer consists of the hydrophilic monomer, the first siloxane monomer and the second siloxane monomer.

### <Hydrophilic monomer>

The hydrophilic monomer can be at least one selected from a group consisting of 2-hydroxyethyl methacrylate (HEMA), methyl methacrylate (MMA), methacrylic acid (MAA), N-vinyl pyrrolidone (NVP), N,N-dimethyl-acrylamide (DMA), 4-acryloylmorpholine (AcMO), 2-hydroxyethyl acrylamide (HEAA), glycidyl methacrylate (GMA), glycerol mono-meth acrylate (GMMA), acrylic acid (AA), N,N-di(methyl meth acryl-amide) (DMA), hexafluoroisopropyl methacrylate (HFMA), N-vinyl-N-methyl acetamide, glycine vinyl carbonate, 2-methacryloyloxyethyl phosphorylcholine, and 2-hydroxy-butyl methacrylate.

The blue light blocking compound can be in a range of about 0.1 wt% to about 30 wt% based on the total weight of the blue light blocking composition, but the present disclosure is not limited thereto. The content of the blue light blocking compound can be adjusted according to the required blue light blocking rate of blue light blocking product.

### <First siloxane monomer>

The first siloxane monomer contains a single methacryloyl group. The methacryloyl group can be represented as CH₂=C(CH₃)CO-; or alternatively, the methacryloyl group can be represented as

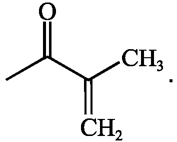

The first siloxane monomer can be at least one selected from a group consisting of the following formula A to formula H.

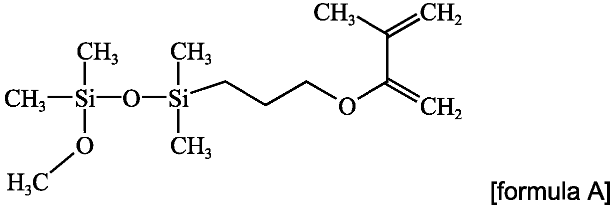

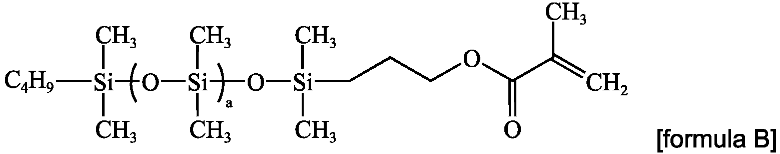

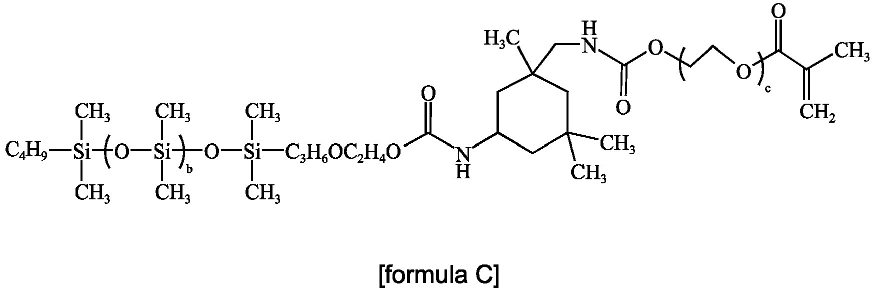

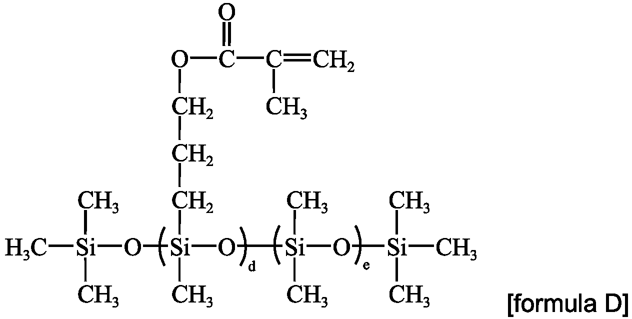

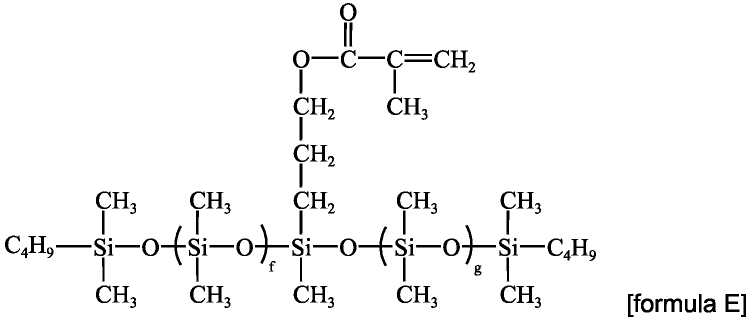

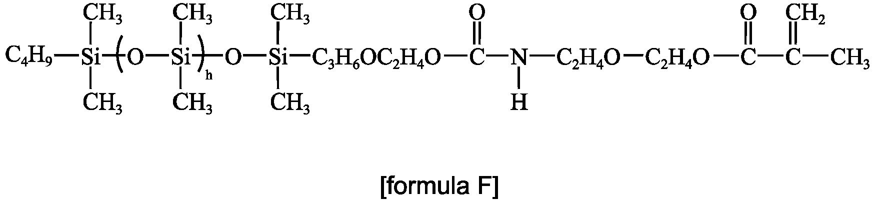

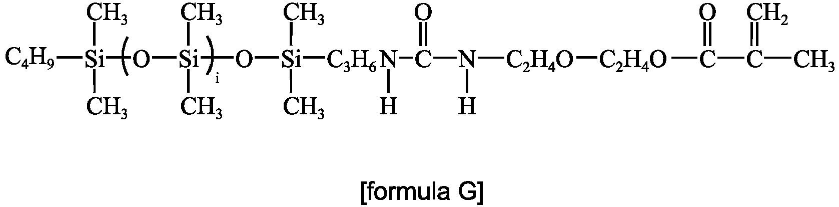

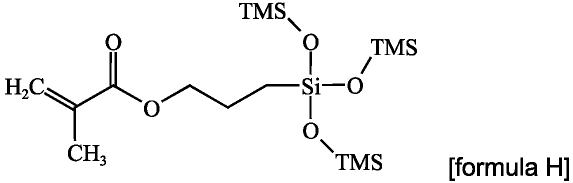

In the formula B, a = 4~80. In the formula C, b = 4~80 and c = 3~40. In the formula D, d = 2~40 and e = 2~40. In the formula E, f = 2~40 and g = 2~40. In the formula F, h = 4~80. In the formula G, i = 4~80. In the formula H, TMS is the abbreviation of trimethylsiloxy.

### <Second siloxane monomer>

The second siloxane monomer contains two methacryloyl groups. The methacryloyl group can be represented as CH₂=C(CH₃)CO-; or alternatively, the methacryloyl group can be represented as

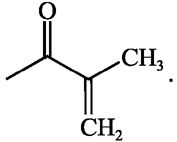

The second siloxane monomer can be at least one selected from a group consisting of the following formula I to formula K.

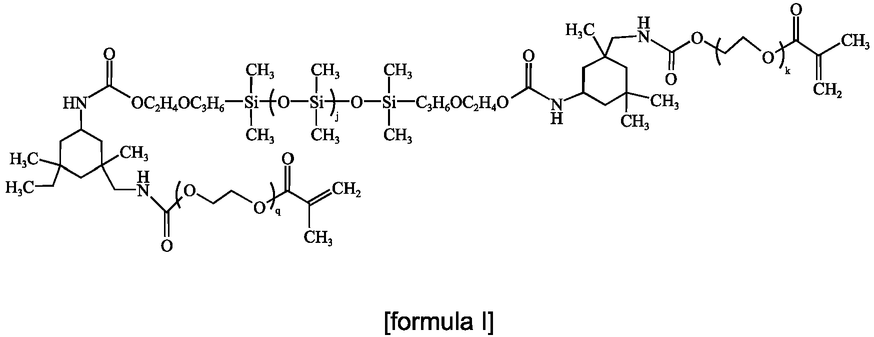

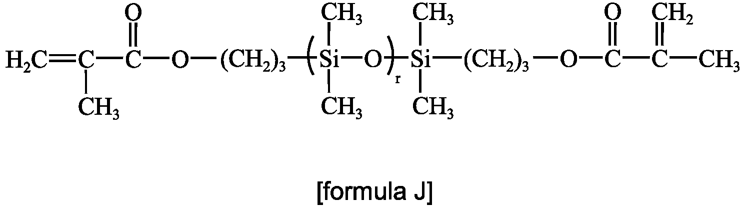

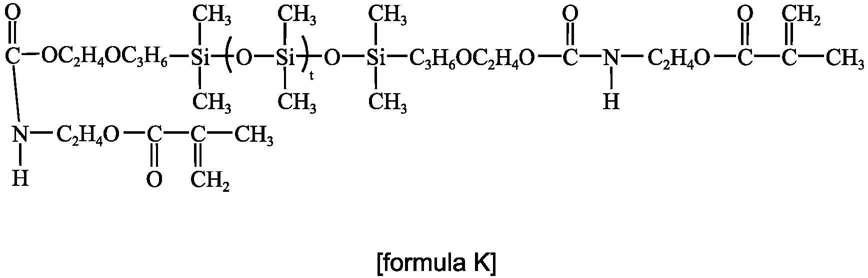

In the formula I, j = 4~80, k = 1~10, and q = 1~10. In the formula J, r = 4~80. In the formula K, t = 4~80.

In an embodiment, the first siloxane monomer can be in a range of 50 wt% to 91 wt%, and the second siloxane monomer can be in a range of 9 wt% to 50 wt% based on the total weight of the first siloxane monomer and the second siloxane monomer. In an embodiment, the first siloxane monomer can be in a range of 60 wt% to 91 wt%, and the second siloxane monomer can be in a range of 9 wt% to 40 wt% based on the total weight of the first siloxane monomer and the second siloxane monomer. In an embodiment, the first siloxane monomer can be in a range of 70 wt% to 91 wt%, and the second siloxane monomer can be in a range of 9 wt% to 30 wt% based on the total weight of the first siloxane monomer and the second siloxane monomer.

The blue light blocking compound can be in a range of about 0.01 wt% to about 5 wt% based on the total weight of the blue light blocking composition (the polymerizable monomer in the blue light blocking composition consists of the hydrophilic monomer, the first siloxane monomer and the second siloxane monomer), but the present disclosure is not limited thereto. The content of the blue light blocking compound can be adjusted according to the required blue light blocking rate of blue light blocking product.

In an embodiment, the blue light blocking composition can further include an ultraviolet absorbing agent, and/or an initiator. The initiator can be a thermal initiator or a photoinitiator.

The ultraviolet absorbing agent can be at least one selected from a group consisting of 2-[3-(2H-benzotriazol-2-yl)-4-hydroxyphenyl]ethyl-2-methacrylate, 2-(4-benzyl-3-hydroxyphenoxy)ethyl acrylate, and N-(4-hydroxy-3(5-methoxy-2H-benzo[d][1,2,3]triazol-2-yl)phenyl)methacrylami de. In an embodiment, the ultraviolet absorbing agent is 2-[3-(2H-benzotriazol-2-yl)-4-hydroxyphenyl]ethyl-2-methacrylate.

The ultraviolet absorbing agent can be in a range of about 0.5 wt% to about 2.5 wt% based on the total weight of the blue light blocking composition.

The thermal initiator can be at least one selected from a group consisting of azobisisobutyronitrile, 2,2'-Azo-bis-(2-methylbutyronitrile), and azobisisoheptanenitrile. In an embodiment, the thermal initiator is azobisisobutyronitrile.

The photoinitiator can be at least one selected from a group consisting of bis(2,4,6-trimethyl benzyl)phenyl-phosphine oxide, benzoin diethyl ether, phenyl dimethyl ketal, α,α-diethoxyacetophenone, and 2-hydroxy-2-methyl-1-phenyl-1-propanone.

The initiator can be in a range of about 0.45 wt% to about 0.75 wt% based on the total weight of the blue light blocking composition.

The above components can be mixed in a specific ratio to form the blue light blocking composition for manufacturing an ophthalmic device. In an embodiment, the blue light blocking composition of the present disclosure may be put into a contact lens mold, and components in the blue light blocking composition undergo a curing reaction (thermal curing reaction or photo curing reaction) through heating or irradiating light to form an ophthalmic device. The heating temperature may be between about 30 °C and about 150 °C, and the heating time may be between about 1 hour and 12 hours. In an embodiment, the reaction conditions can be heating at 30-70 °C for 0-2 hours, heating at 70-100 °C for 2-4 hours, and heating at 100-150 °C for 4-12 hours. In an embodiment, a hydration process and/or a sterilization process can be performed after the curing reaction. The hydration process may include soaking the ophthalmic device in alcohol and pure water, and then put the ophthalmic device in a buffer solution to be equilibria.

The blue light blocking compound of the present disclosure is compatible with various polymerization reactions in different formulations. In an embodiment, the blue light blocking compound of the present disclosure is compatible with various polymerization reactions for manufacturing ophthalmic devices, such as various polymerization reactions for manufacturing hydrogel contact lenses and various polymerization reactions for manufacturing silicone hydrogel contact lenses.

The present disclosure will be explained in further detail with reference to the examples. However, the present disclosure is not limited to these examples.

### [Miscibility and reactivity evaluation]

The blue light blocking compound of Example 1 has the structure represented by the above formula 1-1. The blue light blocking compound of Example 2 has the structure represented by the above formula 1-2. The blue light blocking compound of Example 3 has the structure represented by the above formula 1-3.

The blue light blocking compound of Comparative Example 1 has a structure represented by the following formula 8.

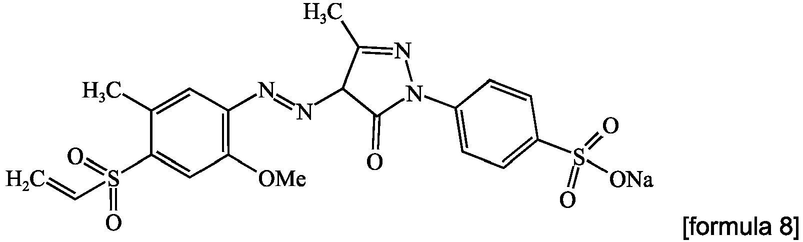

The blue light blocking compounds of Examples 1 to 3 and Comparative Example 1 are dissolved in 2-hydroxyethyl methacrylate (represented by HEMA in the table, which is also the hydrophilic monomer used to manufacture the ophthalmic device) and a silicone hydrogel contact lens composition for observing the miscibility, and the results are shown in the following TABLE 1. The higher the value in the TABLE 1, the greater the miscibility. The silicone hydrogel contact lens composition used in the miscibility evaluation includes a polymerizable monomer, and the polymerizable monomer includes a hydrophilic monomer, a first siloxane monomer and a second siloxane monomer.

**[TABLE 1]**

| | Example 1 | Example 2 | Example 3 | Comparative Example 1 |
|---|---|---|---|---|
| HEMA | 25 % | 67 % | 47 % | 5 % |
| silicone hydrogel contact lens composition | 4.0 % | 4.6% | 1.5 % | 0.1% |

As shown in TABLE 1, the miscibility of blue light blocking compounds of Example 1 to 3 and the materials of the ophthalmic devices are significantly greater than the miscibility of blue light blocking compound of Comparative Example 1 and the material of the ophthalmic device. Therefore, the miscibility of the blue light blocking compound and the material of the ophthalmic device can be effectively improved by the blue light blocking compound of the present disclosure. High miscibility means good reactivity in subsequent polymerization reactions, and thus the reactivity can be effectively improved by the blue light blocking compound of the present disclosure.

### [Blue light absorbance evaluation]

Examples 4 and 5: The blue light blocking compounds of Example 1 are added to the silicone hydrogel contact lens compositions in proportions of 0.1% and 1.0% respectively, and the blue light blocking compound compositions are used to manufacture ophthalmic devices by the above method.

Examples 6 and 7: The blue light blocking compounds of Example 2 are added to the silicone hydrogel contact lens compositions in proportions of 0.1% and 1.0% respectively, and the blue light blocking compound compositions are used to manufacture ophthalmic devices by the above method.

Examples 8 and 9: The blue light blocking compounds of Example 3 are added to the silicone hydrogel contact lens compositions in proportions of 0.1% and 1.0% respectively, and the blue light blocking compound compositions are used to manufacture ophthalmic devices by the above method.

Comparative Examples 2 and 3: The blue light blocking compounds of Comparative Example 1 are added to the silicone hydrogel contact lens compositions in proportions of 0.1% and 1.0% respectively, and the blue light blocking compound compositions are used to manufacture ophthalmic devices by the above method.

The ophthalmic devices of Examples 4 to 9 and Comparative Examples 2 and 3 are subjected to a blue light absorbance evaluation. The results are shown in the following TABLE 2. The blue light absorbance is measured by absorption spectrum with UV-VIS spectrophotometer (SHIMADZU UV-2600). The blue light absorbance represents the amount of blue light with a wavelength between 380 nm and 460 nm that are absorbed by silicone hydrogel contact lens.

**[TABLE 2]**

| | Example | | | | | | Comparative Example | |
|---|---|---|---|---|---|---|---|---|
| | 4 | 5 | 6 | 7 | 8 | 9 | 2 | 3 |
| Addition amount (%) | 0.1 | 1.0 | 0.1 | 1.0 | 0.1 | 1.0 | 0.1 | 1.0 |
| blue light | 8.3 | 35.5 | 9.5 | 35.2 | 8.0 | 26.0 | 7.8 | × |
| absorbance (%) | | | | | | | | |

As shown in TABLE 2, the blue light absorbance of Comparative Example 3 cannot be measured because the ophthalmic device cannot be formed. The reason why it cannot form an ophthalmic device in Comparative Example 3 is that the miscibility of the blue light blocking compound of Comparative Example 1 and the silicone hydrogel contact lens compositions is poor, and precipitation occurs when the addition amount of the blue light blocking compound is 1.0%. As shown in TABLE 2, the blue light absorbance of Examples 4, 6 and 8 are all higher than the blue light absorbance of Comparative Example 2, which means that the blue light blocking compound of the present disclosure has the blue light blocking effect. In addition, in the case of the same addition amount, the blue light blocking effect of the blue light blocking compound of the present disclosure is higher than that of the blue light blocking compound of the Comparative Example. It can be seen from the blue light absorbance of Examples 4, 6 and 8 and Comparative Example 2 that the blue light blocking compound of the present disclosure can obtain better blue light blocking effects at a lower addition amount, which can reduce costs and improve appearance of the ophthalmic device. Moreover, the blue light absorbance of Example 5 is higher than that of Example 4, the blue light absorbance of Example 7 is higher than that of Example 6, and the blue light absorbance of Example 8 is higher than that of Example 6, which means that the blue light absorbance of the blue light blocking compound of the present disclosure increases as the addition amount increases. Moreover, as the addition amount increases, the blue light blocking compound still shows good miscibility.

Furthermore, the blue light blocking compounds of Example 1 can be added to the silicone hydrogel contact lens compositions in proportions of 1.5% and 2% respectively, and the blue light blocking compound compositions are used to manufacture ophthalmic devices by the above method; the ophthalmic devices are subjected to the above blue light absorbance evaluation, and the blue light absorbance of the ophthalmic devices are 56.1 % and 65.3 % respectively, which means that the blue light absorbance of the blue light blocking compound of the present disclosure increases as the addition amount increases. The addition amount of the blue light blocking compound of the present disclosure can be much higher than the addition amount of the blue light blocking compound of Comparative Example to obtain better blue light blocking effects (it can be seen from TABLE 2 that the blue light blocking compound of Comparative Example 1 can only be used in an addition amount of less than 1.0%).

The blue light blocking compound of the present disclosure has good miscibility so that the blue light blocking compound can be evenly dispersed in the material of the ophthalmic device and the use of chemicals that help the blue light blocking compound to be evenly dispersed can be eliminated or reduced. Therefore, the present disclosure can reduce the use of chemicals, simplify the manufacturing process, and reduce the risk of chemical residues in final products (such as blue light blocking ophthalmic devices, blue light blocking coatings and blue light blocking films). In addition, high miscibility means good reactivity in subsequent polymerization reactions and higher-quality ophthalmic devices can be obtained.

While the disclosure has been described by way of example and in terms of the exemplary embodiment(s), it is to be understood that the disclosure is not limited thereto. On the contrary, it is intended to cover various modifications and similar arrangements and procedures, and the scope of the appended claims therefore should be accorded the broadest interpretation so as to encompass all such modifications and similar arrangements and procedures.

## Claims

1. A blue light blocking compound represented by the following formula 1, 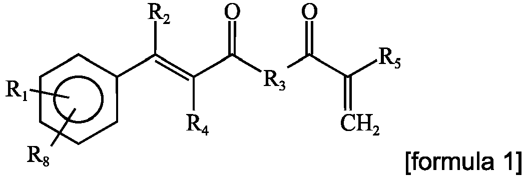 wherein in the formula 1,
R₁ and R₈ are each independently H, a substituted or unsubstituted alkyl group, a substituted or unsubstituted alkenyl group, halogen, N(R^{1a})₂, OR^{1b}, SR^{1c}, SO₂R^{1d}, COR^{1e}, NO₂, CN, SOR^{1h}, a five-membered heterocycle, a six-membered heterocycle, or a fused ring structure formed by connecting R₁ and R₈, wherein R^{1a} and R^{1b} are each independently H, a substituted or unsubstituted alkyl group, a substituted or unsubstituted alkenyl group, or a substituted or unsubstituted phenyl group,
R^{1d} is a substituted or unsubstituted alkyl group, a substituted or unsubstituted alkenyl group or OH,
R^{1e} is H, OH, a substituted or unsubstituted alkyl group, a substituted or unsubstituted alkenyl group or -O-R^{1f},
R^{1c}, R^{1f} and R^{1h} are each independently a substituted or unsubstituted alkyl group, or a substituted or unsubstituted alkenyl group,
R₂ is H or a substituted or unsubstituted C₁~C₄ alkyl group,
R₃ is X-(CₙH₂ₙOₘ)ₚ, wherein X is O, NH, S or SO₂, m is 1 to 4, n is 1 to 4, p is 1 to 4,
R₄ is CN or C(O)R₆, wherein R₆ is H, a substituted or unsubstituted alkyl group, a substituted or unsubstituted alkenyl group, R₃-C(O)-CCH₂R₅ or O-R^{1g},
R^{1g} is a substituted or unsubstituted C₁~C₄ alkyl group, or a substituted or unsubstituted C1~C4 alkenyl group,
R₅ is H or a substituted or unsubstituted C₁~C₄ alkyl group.

2. The blue light blocking compound according to claim 1, wherein X is O or NH, m is 1 to 3, n is 1 to 3, and p is 1 to 3.

3. The blue light blocking compound according to claim 1, wherein X is O or NH, m is 1 to 2, n is 1 to 2, and p is 1 to 2.

4. A blue light blocking composition, comprising:
a blue light blocking compound represented by the following formula 1; and
a polymerizable monomer, 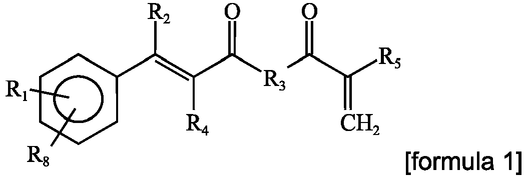 wherein in the formula 1,
R₁ and R₈ are each independently H, a substituted or unsubstituted alkyl group, a substituted or unsubstituted alkenyl group, halogen, N(R^{1a})₂, OR^{1b}, SR^{1c}, SO₂R^{1d}, COR^{1e}, NO₂, CN, SOR^{1h}, a five-membered heterocycle, a six-membered heterocycle, or a fused ring structure formed by connecting R₁ and R₈, wherein R^{1a} and R^{1b} are each independently H, a substituted or unsubstituted alkyl group, a substituted or unsubstituted alkenyl group, or a substituted or unsubstituted phenyl group,
R^{1d} is a substituted or unsubstituted alkyl group, a substituted or unsubstituted alkenyl group or OH,
R^{1e} is H, OH, a substituted or unsubstituted alkyl group, a substituted or unsubstituted alkenyl group or -O-R^{1f},
R^{1c}, R^{1f} and R^{1h} are each independently a substituted or unsubstituted alkyl group, or a substituted or unsubstituted alkenyl group,
R₂ is H or a substituted or unsubstituted C₁~C₄ alkyl group,
R₃ is X-(CₙH₂ₙOₘ)ₚ, wherein X is O, NH, S or SO₂, m is 1 to 4, n is 1 to 4, p is 1 to 4,
R₄ is CN or C(O)R₆, wherein R₆ is H, a substituted or unsubstituted alkyl group, a substituted or unsubstituted alkenyl group, R₃-C(O)-CCH₂R₅ or O-R^{1g}, R^{1g} is a substituted or unsubstituted C₁~C₄ alkyl group, or a substituted or unsubstituted C₁~C₄ alkenyl group,
R₅ is H or a substituted or unsubstituted C₁~C₄ alkyl group.

5. The blue light blocking composition according to claim 4, wherein the polymerizable monomer comprises a hydrophilic monomer selected from a group consisting of 2-hydroxyethyl methacrylate (HEMA), methyl methacrylate (MMA), methacrylic acid (MAA), N-vinyl pyrrolidone (NVP), N,N-dimethyl-acrylamide (DMA), 4-acryloylmorpholine (AcMO), 2-hydroxyethyl acrylamide (HEAA), glycidyl methacrylate (GMA), glycerol mono-meth acrylate (GMMA), acrylic acid (AA), N,N-di(methyl meth acryl-amide) (DMA), hexafluoroisopropyl methacrylate (HFMA), N-vinyl-N-methyl acetamide, glycine vinyl carbonate, 2-methacryloyloxyethyl phosphorylcholine, and 2-hydroxy-butyl methacrylate.

6. The blue light blocking composition according to anyone of the claims 4 to 5, wherein the polymerizable monomer comprises at least one siloxane monomer.

7. The blue light blocking composition according to anyone of the claims 4 to 6, further comprising a thermal initiator or a photoinitiator.

8. A use of a blue light blocking compound according to any one of claims 1 to 3 in manufacturing an ophthalmic device.

9. A use of a blue light blocking composition in manufacturing an optical film, a screen protector, a display or an ophthalmic device, wherein the blue light blocking composition comprises:
a blue light blocking compound represented by the following formula 1; and
a polymerizable monomer, 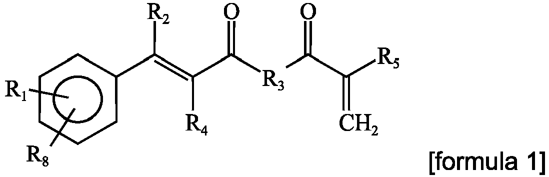 wherein in the formula 1,
R₁ and R₈ are each independently H, a substituted or unsubstituted alkyl group, a substituted or unsubstituted alkenyl group, halogen, N(R^{1a})₂, OR^{1b}, SR^{1c}, SO₂R^{1d}, COR^{1e}, NO₂, CN, SOR^{1h}, a five-membered heterocycle, a six-membered heterocycle, or a fused ring structure formed by connecting R₁ and R₈, wherein R^{1a} and R^{1b} are each independently H, a substituted or unsubstituted alkyl group, a substituted or unsubstituted alkenyl group, or a substituted or unsubstituted phenyl group,
R^{1d} is a substituted or unsubstituted alkyl group, a substituted or unsubstituted alkenyl group or OH,
R^{1e} is H, OH, a substituted or unsubstituted alkyl group, a substituted or unsubstituted alkenyl group or -O-R^{1f},
R^{1c}, R^{1f} and R^{1h} are each independently a substituted or unsubstituted alkyl group, or a substituted or unsubstituted alkenyl group,
R₂ is H or a substituted or unsubstituted C₁~C₄ alkyl group,
R₃ is X-(CₙH₂ₙOₘ)ₚ, wherein X is O, NH, S or SO₂, m is 1 to 4, n is 1 to 4, p is 1 to 4,
R₄ is CN or C(O)R₆, wherein R₆ is H, a substituted or unsubstituted alkyl group, a substituted or unsubstituted alkenyl group, R₃-C(O)-CCH₂R₅ or O-R^{1g}, R^{1g} is a substituted or unsubstituted C₁~C₄ alkyl group, or a substituted or unsubstituted C₁~C₄ alkenyl group,
R₅ is H or a substituted or unsubstituted C₁~C₄ alkyl group.

10. A method for preparing a blue light blocking compound according claim 1, comprising:
providing a compound represented by the following formula 2;
hydrolyzing the compound to obtain an intermediate product; and
esterifying the intermediate product to obtain the blue light blocking compound according to claim 1, 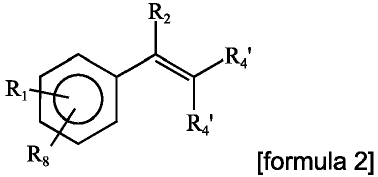 wherein in the formula 2,
R₁ and R₈ are each independently H, a substituted or unsubstituted alkyl group, a substituted or unsubstituted alkenyl group, halogen, N(R^{1a})₂, OR^{1b}, SR^{1c}, SO₂R^{1d}, COR^{1e}, NO₂, CN, SOR^{1h}, a five-membered heterocycle, a six-membered heterocycle, or a fused ring structure formed by connecting R₁ and R₈, wherein R^{1a} and R^{1b} are each independently H, a substituted or unsubstituted alkyl group, a substituted or unsubstituted alkenyl group, or a substituted or unsubstituted phenyl group,
R^{1d} is a substituted or unsubstituted alkyl group, a substituted or unsubstituted alkenyl group or OH,
R^{1e} is H, OH, a substituted or unsubstituted alkyl group, a substituted or unsubstituted alkenyl group or -O-R^{1f},
R^{1c}, R^{1f} and R^{1h} are each independently a substituted or unsubstituted alkyl group, or a substituted or unsubstituted alkenyl group,
R₂ is H or a substituted or unsubstituted C₁~C₄ alkyl group,
R₄' is CN or C(O)R₆', wherein R₆' is H, a substituted or unsubstituted alkyl group, a substituted or unsubstituted alkenyl group, or O-R^{1j}, R^{1j} is a substituted or unsubstituted C₁~C₄ alkyl group, or a substituted or unsubstituted C₁~C₄ alkenyl group.
